(19) **Europäisches Patentamt**
**European Patent Office**
**Office européen des brevets**

(11)  **EP 2 456 547 B2**

(12)  # NEW EUROPEAN PATENT SPECIFICATION
After opposition procedure

(45) Date of publication and mention
of the opposition decision:
**06.03.2024  Bulletin 2024/10**

(45) Mention of the grant of the patent:
**04.06.2014  Bulletin 2014/23**

(21) Application number: **10736633.8**

(22) Date of filing: **23.07.2010**

(51) International Patent Classification (IPC):
**B01F 27/111** $^{(2022.01)}$   **B01F 27/192** $^{(2022.01)}$
**C12M 1/02** $^{(2006.01)}$   **C12M 1/06** $^{(2006.01)}$

(52) Cooperative Patent Classification (CPC):
**C12M 27/08; B01F 27/1111; B01F 27/192;**
B01F 27/111; B01F 27/113; B01F 27/1152

(86) International application number:
**PCT/EP2010/004520**

(87) International publication number:
**WO 2011/009625 (27.01.2011 Gazette 2011/04)**

(54)  **STIRRER SYSTEM**

RÜHRSYSTEM

Système de malaxage multiple

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority:  **24.07.2009  EP 09009639**

(43) Date of publication of application:
**30.05.2012  Bulletin 2012/22**

(73) Proprietor: **F. Hoffmann-La Roche AG**
**4070 Basel (CH)**

(72) Inventors:
- **ALISCH, Alexander**
**82377 Penzberg (DE)**
- **JENZSCH, Marco**
**82377 Penzberg (DE)**
- **POHLSCHEIDT, Michael**
**Carlsbad, CA 92011 (US)**
- **THIELE, Joerg**
**82404 Sindelsdorf (DE)**
- **WALLERIUS, Claus**
**82377 Penzberg (DE)**

(74) Representative: **Bösl, Raphael Konrad**
**Patentanwälte Isenbruck Bösl Hörschler PartG
mbB**
**Eastsite One**
**Seckenheimer Landstraße 4**
**68163 Mannheim (DE)**

(56) References cited:
JP-A- 349 007 160    JP-U- 359 070 499
US-A- 4 438 074     US-A- 4 779 990
US-A- 5 198 156     US-A- 5 972 661
US-A1- 2004 087 814   US-A1- 2004 234 435
US-B1- 6 250 796

- **NIENOW A W: "AGITATORS FOR MYCELIAL
FERMENTATIONS" TRENDS IN
BIOTECHNOLOGY, ELSEVIER PUBLICATIONS,
CAMBRIDGE, GB, vol. 8, no. 8, 1 August 1990
(1990-08-01), pages 224-233, XP000142634 ISSN:
0167-7799**

**EP 2 456 547 B2**

## Description

The present invention concerns a method for culturing mammalian cells and a method for producing a polypeptide as specified in the claims wherein a stirrer system and a cultivation vessel is applied wherein the stirrer system is consisting of two radially-conveying stirrer elements and one axially-conveying stirrer element arranged above one another on a vertical stirrer shaft, wherein the axially-conveying stirrer element is arranged above the radially-conveying stirrer elements. Such a stirrer system achieves among others a gentler and better mixing of the culture medium for the culture of mammalian cells.

## Technological background

The production of recombinant proteins, vaccines and antibodies with the aid of prokaryotic and eukaryotic cells plays an essential role in modem pharmaceutical production. To produce complex post-translationally modified proteins and antibodies animal derived cells are primarily used. However, the use of animal derived cells sets high demands for the fermentation process due to the specific characteristics of these cells such as e.g. the culture medium, the sensitivity towards limitation and inhibitions (for example by lactate, $CO_2$, ammonium etc.), the sensitive outer membrane (shear stress), the low specific rates and the sensitivity towards variations in the culture conditions (e.g. due to local inhomogeneities, pH variations, $pO_2$ variations etc.). These properties have to be taken into consideration when designing bioreactors and for process control.

In recent years various types of reactors for culturing cells have been developed. Irrespective of the type, the reactor must be able to fulfill the following basic technical functions: adequate suspension as well as homogenization, adequate material and heat transport as well as a minimal shear stress on the cells. The stirred-tank reactor is especially suitable for industrial use. In this reactor the necessary energy for fulfilling the basic functions is introduced by mechanical stirring.

It is known from the literature that mammalian cells are much more sensitive to shear forces than microorganisms. This is usually attributed to the lack of a cell wall (see e.g. Glacken, M.W., et al., Trends Biotechnol. 1 (1983) 102-108; van der Pol, L.A. and Tramper, J. Enzyme Microb. Technol. 17 (1995) 401-407; Nienow, A.W., Cytotechnol. 50 (2006) 9-33; Cervantes, M.I., et al., Chem. Eng. Sci. 61 (2006) 8075-8084; Frahm, B., et al., Chem. Ing. Tech. 79 (2007) 1052-1058). The shear sensitivity is the reason for considerable difficulties in the technical handling of suspension culture bioreactors. Stirred tank reactors are usually used in biotechnology production processes to feed the cells as these can be more flexibly controlled and used with regard to the required process conditions. In addition to the geometry of the systems and the rheology of the stirred material, the above-mentioned basic functions are primarily fulfilled by the stirrer systems that are used.

The main reasons for shear stress on the cells to be cultured are among others the shear forces generated by the stirrer system. These have an intense effect especially in the region of the stirrer blades as well as that of the baffles. According to the theory of Kolmogorov the macro-vortices generated by the stirrers decompose by energy dissipation to form small micro-vortices. When their size becomes the same as the size of the cells to be fermented, the cells can be damaged (Cherry, R.S. and Papoutsakis, E.T., Biotechnol. Bioeng. 32 (1988) 1001-1014; Papoutsakis, E.T. and Kunas, K.T., in "Advances in animal cell biology and technology for bioprocesses", Spier, R.E., et al. (Eds) Butterworth, Sevenoaks, Kent, UK (1989) 203-211; Kunas, K.T. and Papoutsakis, E.T., Biotech. Bioeng. 36 (1990) 476-483; Zhang, Z.B. and Thomas, C.R., Gen. Eng. Biotechnol. 13 (1993) 19-29; Nienow, A.W., Cytotechnol. 50 (2006) 9-33).

Recommendations have been made for the use of stirrers in animal suspension cell cultures (see e.g. Feder, J. and Tolbert, W.R., Sci. Am. 248 (1983) 24-31; stirrer manufacturers for example Ekato, Chemineer, Bioengineering, Zeta) which emphasize the generation of low shear flows as an important criterion. Hence a large number of low shear force stirrers has been developed to reduce damage (e.g. "Elephant Ear Impeller", or "Max Flow Impeller").

In an online article ("Axial flow down-pumping agitators in biological processes", April 2009, http://www.postmixing. com/mixing%20forum/Micro/Liq-Solid-Gas/down-pumpers.htm) it is shown that a stirrer combination consisting of different stirrers results in an improved mass transfer. With regard to animal cells, the use of three axial transporters is recommended to achieve the best results in cell culture. Fujasova, M., et al., (Chem. Eng. Sci. 62 (2007) 1650-1669) report correlations in the mass transfer for multistage stirrers. Mass transfer between gas phase and liquid phase using a three stage stirrer systems in a bioreactor is reported by Puthli et al. (Puthli, M.S., et al., Biochem. Eng. J. 23 (2005) 25-30).

The use of a fermenter for culturing bacterial cells with a vertical rotary shaft to which preferably two or three Rushton stirrers are attached is reported in US 5,633,165. Nienow, A.W., (Cytotechnol. 50 (2006) 9-33) recommends a reactor system for animal cell culture with an H/D ratio of 1 to 1.3 and the use of two downwards conveying hydrofoil stirrers (axial transporters) with an aerator which is mounted below the lowest stirrer.

In US 2004/0234435 an apparatus for and method of producing aromatic carboxylic acids is reported. A continuous polymerization reactor is reported in US 4,438,074. In US 2004/0087814 an agitation system for alkyl benzene oxidation reactors is reported. A mixing system is reported in US 5,972,661. In US 6,250,769 an agitation apparatus with static mixer or swirler means is reported. Agitators are reported in US 5,198,156. In US 4,779,990 an impeller apparatus is reported.

## Summary of the invention

The present invention concerns a method for culturing mammalian cells and a method for producing a polypeptide as specified in the claims. In comparison to the stirrers that are usually used to culture shear-sensitive animal cells, the stirrer system as reported herein can achieve a more rapid mixing of the culture medium (for example in order to introduce correcting agents such as acids or bases via the liquid surface) without exposing the cells in the cultivation medium to high shear stress.

In one embodiment the stirrer system is consisting of 3 to 5 conveying elements. In another embodiment the stirrer system is consisting of 3 or 4 conveying elements. In a further embodiment the conveying elements of the stirrer system are arranged at a certain distance above each other on a vertical shaft. In yet another embodiment the conveying elements are arranged on the shaft with the same distance to each other. In yet another embodiment the distance between the conveying elements is between one and two conveying element diameters d. In one embodiment the conveying elements of the stirrer system are arranged on a vertical shaft whereby the uppermost conveying element is at a defined distance from the bottom conveying element and is at a sufficient distance from the liquid surface of the cultivation medium when the stirrer system is operated in a cultivation vessel filled with cultivation medium whereby the stirrer system ensures mixing of the cultivation medium. In one embodiment the distance from the surface of the cultivation medium is the same as the distance between the uppermost stirrer element and the stirrer element that is second from the top. In one embodiment all conveying elements have the same diameter d.

According to the claimed method the stirrer system is consisting of two radially-conveying elements and one axially conveying element, wherein the axially-conveying element is arranged on the stirrer shaft above the radially-conveying elements.

In one embodiment the radially-conveying elements have between 2 and 8 stirrer blades and the axially conveying element has between 2 and 10 stirrer blades. In another embodiment the radially-conveying elements have 3 to 6 stirrer blades, and in a further embodiment 6 stirrer blades. In one embodiment the axially conveying element has 2 to 6 stirrer blades, and in a further embodiment 4 stirrer blades. In a further embodiment all radially-conveying and all axially-conveying elements have the same number of stirrer blades. In one embodiment all conveying elements have 4 or 6 stirrer blades. In one embodiment the radially-conveying element is a symmetrical radially-conveying element. A "symmetrical radially-conveying element" is an element that has a symmetrical cross-section along the longitudinal axis of the element, i.e. the cross-section along and including the rotation axis is point symmetric and mirror-symmetric.

In one embodiment the ratio of the diameter of the conveying element d to the diameter of the cultivation vessel D when the stirrer system is placed in the cultivation vessel is in the range between 0.2 and 0.8, in another embodiment in the range between 0.3 and 0.6, in a further embodiment in the range between 0.31 and 0.39, or in also an embodiment about 0.34. The ratio of the height of the stirrer blade of the axially-conveying element hB to the width of the stirrer blades of the radially-conveying element b is in the range between 0.2 and 2.0, in another embodiment in the range between 0.3 and 1.4, or in a further embodiment in the range between 0.4 and 1.0. In a further embodiment the pitch of the stirrer blades of the axially-conveying element is between 10° and 80°, in another embodiment between 24° and 60°, or in a further embodiment between 40° and 50° relative to the shaft axis. In one embodiment all conveying elements have a ratio of the diameter of the conveying element d to the diameter of the cultivation vessel D of from 0.32 to 0.35.

In one embodiment the stirrer system has a Newton number of from 5.5 to 8.0 at a Reynolds number of from $5*10^4$ to $5*10^5$. In another embodiment the stirrer system has a mixing time $\theta_{0.95}$ of about 20 seconds at a power input of about 0.05 W/kg and of about 10 seconds at a power input of about 0.3 W/kg.

Also an aspect as reported herein is a method for producing a polypeptide comprising the steps as specified in the claims.

In one embodiment the purifying is a multistage chromatographic process. In another embodiment the purifying comprises an affinity chromatography, a cation exchange chromatography and an anion exchange chromatography.

Another aspect as reported herein is a method for culturing animal cells, characterized in that the animal cells are cultured in a cultivation vessel comprising a stirrer system as reported herein.

In one embodiment the culturing is a semi-continuous culturing. In a further embodiment the semi-continuous culturing is a dialysis. In one embodiment the polypeptide is an antibody or an antibody derivative.

In one embodiment of the previous aspects the cultivation medium is an aqueous medium which is suitable for the cultivation of mammalian cells. In another embodiment the cultivation medium is a Newtonian liquid. In one embodiment the stirrer system is operated at a power input of from 0.01 W/kg to 1 W/kg. In a further embodiment the stirrer system is operated at a power input of from 0.04 W/kg to 0.5 W/kg. In still another embodiment the flow induced by the stirrer system in the cultivation medium is a turbulent flow. In another embodiment the cultivation medium has a viscosity of 3 mPas*s or less. In another embodiment the viscosity is 2 mPas*s or less.

A further aspect as reported herein is a method for culturing mammalian cells or hybridoma cells for the production of polypeptides or antibodies. In one embodiment the culturing is carried out in a submersed gassed stirred tank reactor. In another embodiment the animal cell is a mammalian cell. In yet a further embodiment the cell is a CHO cell, a BHK cell, an NS0 cell, a COS cell, a PER.C6 cell, a Sp2/0 cell, an HEK 293 cell or a hybridoma cell. In yet a further embodiment the antibody is an antibody against CD19, CD20, CD22, HLA-DR, CD33, CD52, EGFR, G250, GD3, HER2, PSMA, CD56, VEGF, VEGF2, CEA, Lewis Y antigen, IL-6 receptor, or IGF-1 receptor.

## Detailed description of the invention

The invention is defined in the independent claims.

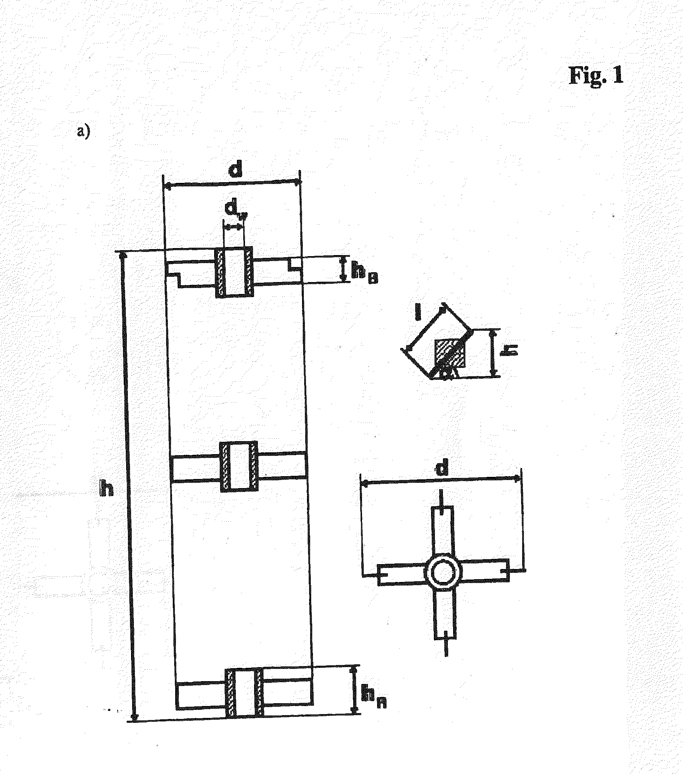

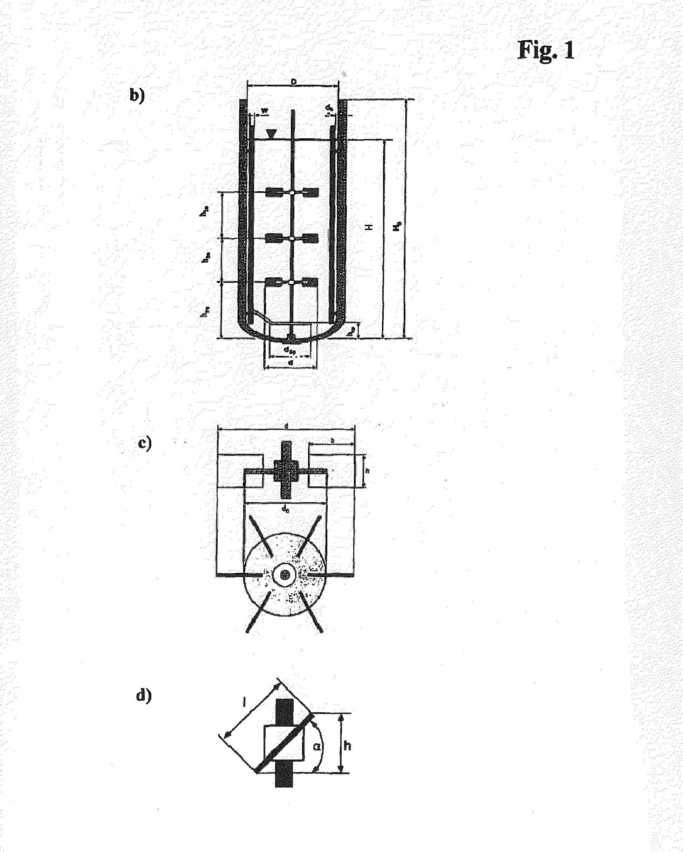

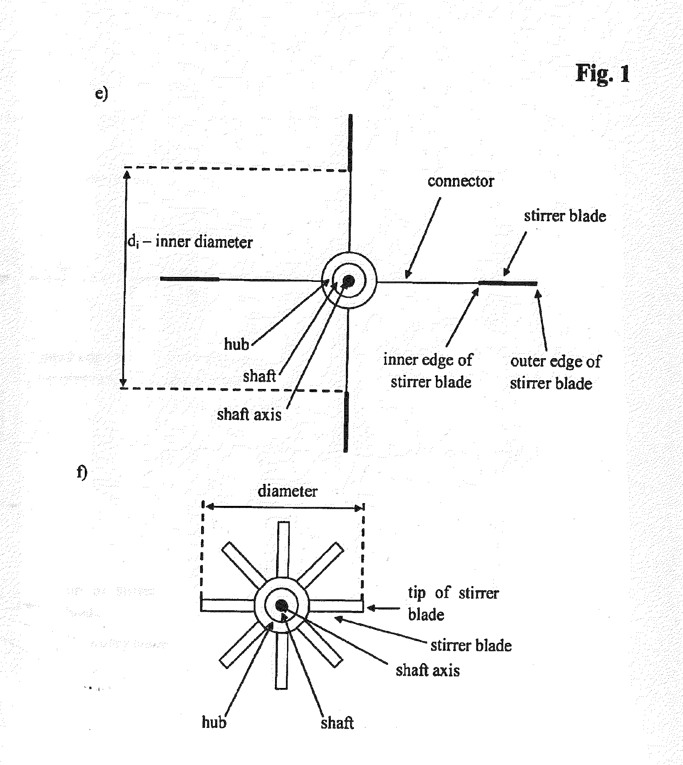

In comparison to stirrer systems that are currently used to culture shear-sensitive animal cells, the stirrer system as reported herein allows a more gentle and more rapid mixing of the cultivation medium. An example of a stirrer system as reported herein is shown in Figure 1. Thus, the stirrer system as reported herein provides for shorter mixing times and also for less stress imparted to the cultivated cells without the need of complex additional components such as baffles, static mixers, swirlers, draft tubes, or other means for directing the flow inside a cultivation vessel.

The terms "element" or "conveying element", which can be used interchangeably, denote a (functional) unit of stirrer blades which are in a fixed spatial configuration relative to one another with regard to distance and angle. A radially-conveying element denotes an element in which the stirrer blades have no pitch with respect to the shaft axis. An axially-conveying element denotes an element in which the stirrer blades have a pitch with respect to the shaft axis. The stirrer blades of the conveying elements are in one embodiment rectangular plates although other geometric forms can be used. The conveying direction of an element is denoted with respect to the rotation axis of the element. The stirrer blades of the conveying elements may not extend from the shaft itself but can be mounted on an arm or an equivalent means on the shaft. Each of the conveying elements consists of a defined number of stirrer blades. Each blade is either directly connected to the rotary shaft or is connected to the rotary shaft via a hub. Each stirrer blade independent of the conveying element has an outer edge and an inner edge. The part of each stirrer blade that has the maximum distance to the shaft is denoted as tip of the blade. Each conveying element has an outer diameter and an inner diameter. For example, the outer diameter of an axially-conveying element is the maximum distance between the tips of opposing stirrer blades and the inner diameter of a radially-conveying element is the minimum distance between inner edges of opposing stirrer blades.

The term "antibody" denotes a protein consisting of one or more polypeptide(s) substantially encoded by immunoglobulin genes. The recognized immunoglobulin genes include the different constant region genes as well as the myriad immunoglobulin variable region genes. Antibodies can exist in a variety of formats, including, for example, Fv, Fab, and F(ab)2 as well as single chains (scFv) or diabodies or triabodies, as monovalent, divalent, trivalent, tetravalent, pentavalent and hexavalent forms, as well as monospecific, bispecific, trispecific or tetraspecific antibodies.

A "polypeptide" is a polymer consisting of amino acids joined by peptide bonds, whether produced naturally or synthetically. Polypeptides of less than about 20 amino acid residues may be referred to as "peptides", whereas molecules consisting of two or more polypeptides or comprising one polypeptide of more than 100 amino acid residues may be referred to as "proteins". A polypeptide may also comprise non-amino acid components, such as carbohydrate groups, metal ions, or carboxylic acid esters. The non-amino acid components may be added by the cell, in which the polypeptide is produced, and may vary with the type of cell. Polypeptides are defined herein in terms of their amino acid backbone structure or the nucleic acid encoding the same. Additions such as carbohydrate groups are generally not specified, but may be present nonetheless.

Submerse gassed cultivations vessels are generally used to culture animal cells for the production of polypeptides or antibodies. One stage or multistage purely axially-conveying or purely radially-conveying stirrer systems are used therein. Herein the term "axially-conveying" denotes that the conveying element generates a flow parallel to the shaft or rotation axis of the element which is directed away from the element. Analogously the term "radially-conveying" denotes that the conveying element generates a flow that is directed away from the stirrer element and that is perpendicular to the shaft or rotation axis of the element. This observation is irrespective of the fact that the reactor has a limited spatial dimension and, thus, the flow is deflected at the reactor walls and, on the other hand, irrespective of the fact that the flow leading to the stirrer element can be different. The shaft and also the shaft axis extend through the longitudinal axis of the cultivation vessel in which the conveying elements or stirrer system is used. The rotary speed n of the element is used as a characteristic velocity and the element diameter d is used as a characteristic length.

An improved stirrer system as reported herein can be provided by using a combination of conveying elements for use in the cultivation of mammalian cells. With the stirrer system as reported herein comparable viabilities, cell densities and product titers can be achieved with at the same time reduced shear stress for the cultured cells. It was found that the stirrer system as reported herein can improve the mixing of the cultivation vessel contents and at the same time retain the achievable cell density e.g. compared to a stirrer system consisting of three axially-conveying elements (such as three inclined-blade stirrers). The stirrer system as reported herein is also particularly suitable for admixing liquids at or from the surface of the cultivation medium for example in order to introduce correcting agents such as e.g. acids, bases, nutrient medium, defoamers or also $CO_2$ or $O_2$ via the liquid surface, and for rapid total mixing of the culture medium when culturing animal cells.

In fluid dynamics the Reynolds's number (Re) describes the ratio of inertial force to inner frictional force in a hydrodynamic system. Therefrom also statements can be made about the degree of turbulence of the moved medium. For stirred liquids the stirrer Reynolds's number is defined in Equation 1 to be

$$Re = \frac{n \cdot d^2}{\nu} = \frac{n \cdot d^2 \cdot \rho}{\eta}$$

(Equation 1)

The Newton number (Ne, also referred to as power number) describes the ratio of resistance force to flow force and is thus a measure for the flow resistance of a stirrer in a stirred material and is described in Equation 2:

$$Ne = \frac{P}{\rho \cdot n^3 \cdot d^5}$$

(Equation 2)

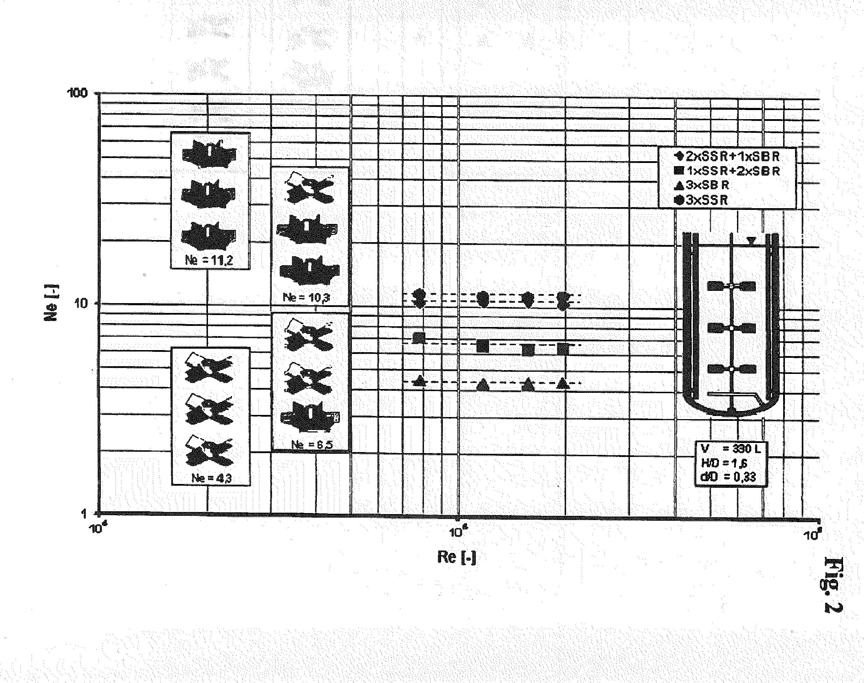

The Ne numbers for different stirrer systems are shown in Figure 2.

Conveying elements with a low Newton number, such as propeller or inclined blade stirrers, convert the power input more efficiently in hydrodynamic output, i.e. fluid motion, than those with a high Newton number, such as Rushton turbines.

A criterion for assessing the stirring processes in a cultivation processes is the mixing time. The "mixing time" of an inhomogeneous liquid-liquid mixture denotes the time which is required to achieve a defined homogeneity in the cultivation medium. Factors influencing the mixing time are the degree of mixing and the site of observation. The degree of mixing in turn depends on the reactor geometry, the stirrer geometry, the rotary frequency of the stirrer, and the substances of the stirred materials. It is important for cultivation processes that, as far as possible, all cells are optimally and uniformly supplied with the necessary substrates (such as nutrient medium, $O_2$) and that metabolites (such as overflow products, $CO_2$) are concomitantly led away. This means that repositories and sinks that may occur spatially as well as temporarily in the cultivation vessel have to be avoided or at least minimized in order to avoid damage to the cells. This can be achieved e.g. by using an improved stirring system for mixing the vessel's contents. The mixing processes can be divided into the sub-processes micro-mixing and macro-mixing. Micro-mixing is defined as the molecular concentration adjustment due to diffusion or microturbulences; in contrast thereto, macro-mixing is defined as the convective coarse mixing caused by the stirrer (see e.g. Houcine, I., et al., Chem. Eng. Technol. 23 (2000) 605-613; Zlokarnik, M., "Rührtechnik Theorie und Praxis", Springer Publishers, Berlin Heidelberg, 1999). The degree of mixing can according to Henzler (Henzler, H.-J., "Homogenisieren: Referenz-Rührsysteme und Methoden zur Erfassung der Homogenisierungseigenschaften von Rührsystemen", GVC Fachausschuß Mischvorgänge, 1998) be defined as follows in Equation 3:

$$X_1 = 1 - \frac{\Delta a}{\overline{a}}$$

(Equation 3).

In this equation $\overline{a}$ corresponds to the concentration of the tracer substance after a theoretically complete mixing and $\Delta a$ corresponds to the maximum difference between the local concentrations of the tracer substance at a time t. In general a degree of mixing of $X_1 = 0.95$ is regarded as sufficient (see Henzler, supra). The mixing coefficient $C_{H\,0.95}$ as described in Equation 4 is based on this degree of mixing and is the product of the mixing time $\theta_{0.95}$ and the rotary speed of the stirrer n that was used. Thus, it corresponds to the number of stirrer revolutions which are required after adding an agent in order to achieve a degree of intermixing of 0.95.

$$C_{H0,95} = \Theta_{0,95} \cdot n \qquad \text{(Equation 4)}.$$

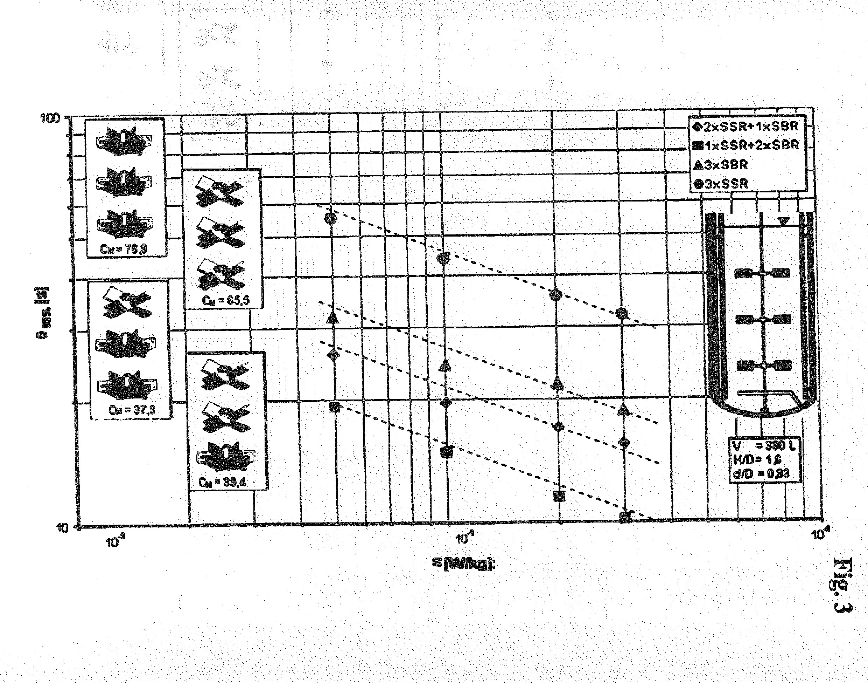

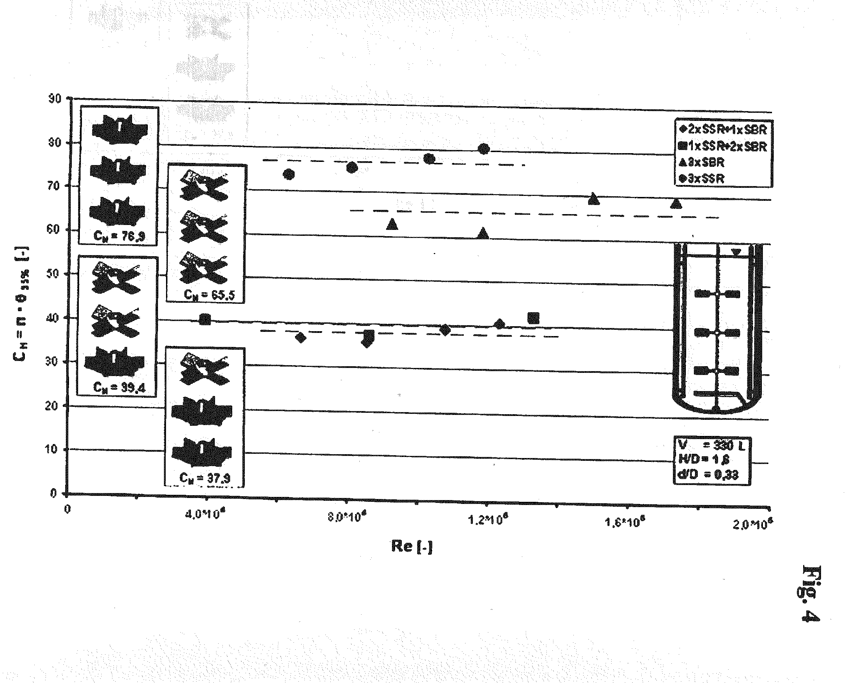

The mixing coefficients shown in Table 1 and in extracts in Figures 3 and 4 resulted from the mixing time investigations using the decolorizing method.

**Table 1:** Mixing coefficients of various stirrers

| Stirrer | H/D | d/D | $C_H(Re)$ |
|---|---|---|---|
| stirrer system with two separate standard disk stirrers (2SSR) | 1.6 | 0.34 | 78 |
| stirrer system with two separate inclined-blade stirrers (2SBR) | 1.6 | 0.34 | 95 |
| stirrer system with three separate standard disk stirrers (3SSR) | 1.6 | 0.33 | 77 |
| stirrer system with three separate inclined-blade stirrers (3SBR) | 1.6 | 0.33 | 65.5 |
| stirrer system with one inclined-blade stirrer and two standard disk stirrers (1SBR + 2SSR) | 1.6 | 0.34 | 38 |
| stirrer system with two inclined-blade stirrers and one standard disk stirrer (2SBR + 1 SSR) | 1.6 | 0.34 | 39 |

In comparison of the configurations the stirrer system as reported herein has an average mixing index of approximately 38 at a constant Reynolds number in comparison, e.g., to a stirrer consisting of three separate inclined-blade stirrers (3SBR) or of three separate standard disk stirrers (3SSR) with an average mixing index of 65.5 and 77, respectively. Thus, in one embodiment of the device as reported herein the ratio of the filling height of the cultivation vessel H to the diameter of the cultivation vessel D is about 1.6. The term "about" denotes that the thereafter following value is no exact value but merely the center value of a range. In one embodiment denotes the term about a range of +/- 25 % around the center value, in a further embodiment of +/- 15 %, and in still another embodiment of +/-10%.

Another process parameter for the cultivation of animal cells is the shear stress applied to the cell in the cultivation medium. Animal cell cultures are, among others, limited by the mechanical and hydrodynamic stress applied to the cells. The stress is, on the one hand, caused by the stirrer itself and, on the other hand, by the bubble aeration of the culture medium (see e.g. Wollny, S. and Sperling, R., Chem. Ing. Tec. 79 (2007) 199-208). For the turbulent flow regions which are mostly predominant in stirred tank vessels, the hydrodynamic stress is given by the Reynolds's stress approach according to Equation 5 (Henzler, H.J. and Biedermann, A., Chem. Ing. Tec. 68 (1996) 1546-1561):

$$\tau_{turb} = \rho \cdot u'^2 \qquad \text{(Equation 5)}.$$

According to Equation 5 the main stress can be deduced as being due to the turbulent velocity fluctuation $u'$ of the fluid elements.

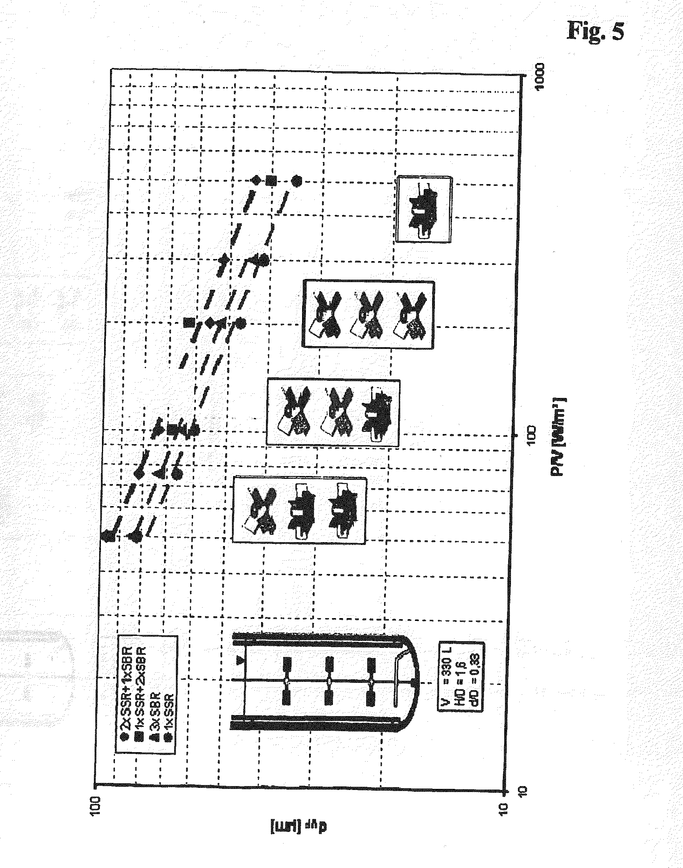

The characterization of shear stress was carried out for the stirrer systems in the non-gassed state. The reference flake diameters $d_{VF}$ describe a relative measure for the prevailing stress and are shown in Figure 5 and Table 2. The higher the reference flake diameter, the lower is the hydrodynamic stress. In this case the sole influence is due to the stirrer system as the aeration is not switched on.

**Table 2:** Examples of reference flake diameters of various stirrer systems at a power input of 100 W/m³.

| Stirrer | H/D | d/D | $d_{VF}(100 \text{ W/m}^3)$ [$\mu$m] |
|---|---|---|---|
| stirrer system with three separate standard disk stirrers (3SSR) | 1.6 | 0.33 | 65 |

(continued)

| Stirrer | H/D | d/D | $d_{VF}$(100 W/m$^3$) [μm] |
|---|---|---|---|
| stirrer system with three separate inclined-blade stirrers (3SBR) | 1.6 | 0.33 | 65 |
| stirrer system with one inclined-blade stirrer and two standard disk stirrers (1SBR + 2SSR) | 1.6 | 0.34 | 75 |
| stirrer system with two inclined-blade stirrers and one standard disk stirrer (2SBR + 1 SSR) | 1.6 | 0.34 | 75 |

In comparison to stirrer systems with only axially-conveying elements, such as for example three inclined-blade stirrers, the stirrer system as reported herein causes considerably less stress, i.e. provides flakes with a bigger reference flake diameter $d_{VF}$.

Figure 2 shows a comparison of Newton numbers from which the non-gassed power inputs can be determined. It can be seen that the stirrer system as reported herein can generate a considerably higher Newton number which confers a gentler and more uniform energy input.

In a direct comparison with various stirrer systems the stirrer system as reported herein exhibits considerable advantages in the mixing (Figures 3 and 4, Table 1) as well as in the generated shear stress (Figure 5, Table 2).

In order to achieve high product titer and a good product quality the operating mode of the cultivation vessel has an important role in addition, e.g., to the cell line development, the media composition and the dimensioning of the cultivation vessel. A distinction can be made between the operating modes batch or batch processes, fed batch or feeding processes, continuous processes with or without cell retention (for example perfusion or chemostat) as well as semi-continuous processes such as e.g. internal or external dialysis.

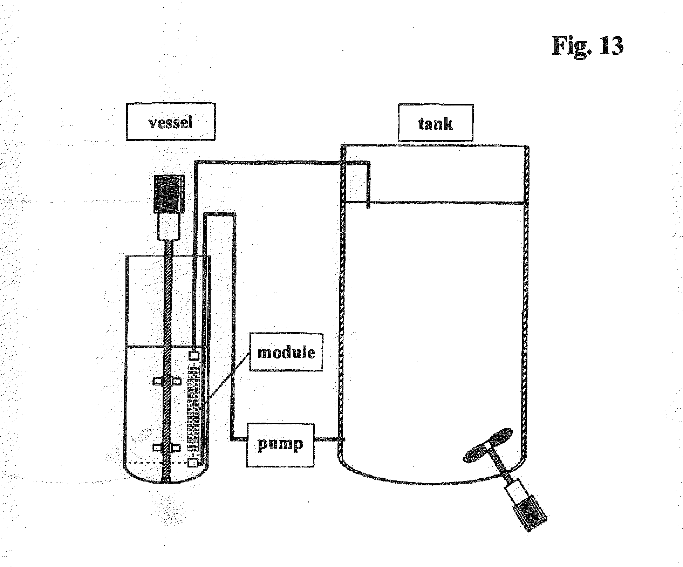

In semi-continuous cultivation processes, such as e.g. external or internal dialysis, substrates are fed to the cultivation vessel across a membrane and at the same time inhibiting components / metabolic products of the cultured cells are lead away. This exchange of material is by diffusion. The main influence factors therefore are the prevailing concentration difference, the membrane material, the membrane surface, the diffusion coefficients of the respective compounds inside the membrane material and the thickness of the phase interface which is determined by the flow against the membrane. When dialysis is employed in high cell density fermentation it is a perfusion-like, semi-continuous process in which a (hollow fiber) dialysis module attached in the reactor provides the exchange area between the cultivation medium and fresh nutrient medium. The nutrient medium is pumped from a storage container through the dialysis module and thereafter returned again into the storage container (for a schematic diagram see Figure 13). The dialysis module can be located outside the reactor (external dialysis) or within the reactor (internal dialysis). The same physical laws apply to both operating modes. Thus, reported herein as one aspect is a device comprising a stirrer system as reported herein and a cultivation vessel and optionally a dialysis module.

The cultivation vessel has an upper portion, a middle portion and a lower portion, wherein the longitudinal axis of the vessel extends from the middle or center of the upper portion to the middle or center of the lower portion. The cylindrical cultivation vessel has a substantially circular cross-section when viewed perpendicular to the longitudinal axis. The upper portion of the cultivation vessel may further comprise a gas discharge outlet means, one or more inlet means, and/or a man hole means for maintenance and cleaning. The lower portion of the cultivation vessel may further comprise one or more liquid media inlet means, one or more liquid media outlet means, and/or a gas inlet means. The middle portion of the cultivation vessel may further comprise a heat exchange jacket mounted to the outside wall of the cultivation vessel. The conveying-elements of the stirrer system are set into rotation by a shaft which is coupled to a suitable mechanism for inducing a rotation thereof. The shaft extends along the longitudinal axis of the cultivation vessel and, thus, the shaft has a vertically oriented axis of rotation. The shaft does not extend to the bottom of the vessel but to a point well above the bottom of the vessel and also well above an optional gas-sparger at the bottom of the cultivation vessel. The shaft is operably coupled to a drive shaft by a suitable coupling mechanism. Beside a means for coupling the shaft to the drive shaft the shaft in addition compromises further means, i.e. at least three means, for individually coupling the conveying-elements to the shaft. The conveying elements of the stirrer system are coupled to the shaft at a position that is/will be below the surface of the cultivation medium in the cultivation vessel once the stirrer system is submersed in the cultivation medium. The surface is determined when the cultivation medium is static, i.e. not being circulated. The cultivation vessel does not contain a draft tube.

In one optional embodiment the cultivation vessel is a baffled vessel. In another embodiment the cultivation vessel comprises two or four baffles. A "baffle" denotes a plate placed inside a cultivation vessel in the same direction as the shaft axis and extending radially into the cultivation vessel towards the agitator. The baffle is generally rectangular in shape. In one embodiment the baffle is placed at a distance $b_d$ to the inner wall of the cultivation vessel. In another embodiment the baffles are spaced equidistally around the circumference of the inside of the cultivation vessel.

In one embodiment the device also comprises a dialysis module. The components of the device are dimensioned in a way that they can exert their intended function, i.e. the cultivation vessel can take up the cultivation medium, the stirrer system can mix the medium and disperse added compound therein and the dialysis module can provide fresh medium and lead away metabolic compounds secreted by the cultivated cell. Thus, the stirrer system has a diameter that allows for an unhampered rotation within the vessel in the presence and absence of the dialysis module. With the device as reported herein a high cell density cultivation as perfusion cultivation or as dialysis cultivation can be carried out advantageously. The culturing is carried out in one embodiment at a rotation speed of the stirrer system at which a Reynolds-number independent constant power input to the cultivation medium can be achieved, i.e. during the culturing a turbulent cultivation medium flow in the cultivation vessel is provided. It is possible with a device as reported herein to cultivate shear sensitive mammalian cells at a low rotation speed of the stirrer system but at the same power input compared to other stirrer systems.

The form of the cultivation vessel is not limited. In one embodiment the cultivation vessel is a cylindrical vessel. In another embodiment the cultivation vessel is a stirred tank reactor. The cultivation vessel may have any dimension. In one embodiment the cultivation vessel has a working volume of from 5 1 to 25,000 1. Components from the fresh nutrient medium diffuse from the interior of the dialysis module through the semi-permeable hollow fiber membrane into the cultivation vessel and at the same time metabolites of the cultivated cells diffuse in the opposite direction from the cultivation vessel into the nutrient medium according to the concentration difference. The aim is to keep the absolute concentration of inhibiting metabolites in the cultivation vessel as low as possible (dilution) and at the same time to maintain the concentration of essential nutrients as long as possible at an optimal level in the culture. This results in improved culture conditions compared to a process without dialysis allowing to achieve higher maximum cell density or product titer.

The transport processes in the dialysis module can be described in an equivalent manner to mass transfer on a gas bubble by the two-film theory in conjunction with the first Fick's law. Thus, assuming linear gradients based on the exchange area $A_H$ of the hollow fiber dialysis module, the effective transferred diffusion flux $J_{eff}$ is according to Equation 6:

$$\overline{J}_{eff,i} = -D_{eff,i} \cdot \overline{A}_H \cdot \frac{c_{2,i} - c_{1,i}}{x_2 - x_1} = -D_{eff,i} \cdot \overline{A}_H \cdot \frac{\Delta c_i}{z_{eff}} \qquad \text{(Equation 6)}.$$

The driving force of diffusion is the concentration difference $\Delta_{Ci}$ between the inside and outside of the dialysis module relative to the effective diffusion path $z_{eff}$. This effective diffusion path is composed of the individual paths through the inner laminar boundary layer on the inner side of the hollow fiber membrane of the dialysis module $\delta_{BI}$, through the hollow fiber membrane itself $\delta_M$ and through the outer laminar boundary layer on the outer side of the hollow fiber membrane in the reactor $\delta_{HI}$. They generally depend on the size and shape of the diffusing molecule, the properties of the surrounding medium and the temperature.

Separate mass transfer coefficients can be defined for the respective individual sections and from the summation of their reciprocals the total mass transfer resistance 1/k is given according to Equation 7:

$$\frac{1}{k} = \frac{1}{k_{HI}} + \frac{1}{k_M} + \frac{1}{k_{BI}} \qquad \text{(Equation 7)}.$$

The transport resistances in the laminar boundary layers on the inner and outer side of the hollow fiber membrane also depend on the flow against the follow fiber membrane. The better, i.e. the more perpendicular, the flow towards the membrane is the narrower the laminar boundary layers become and the lower are the corresponding transport resistances. For a dialysis module in a cultivation vessel a direct dependency of the transport resistance of the outer laminar boundary layer on among others from the following factors exists:

■ the speed of rotation of the stirrer system,

■ the type of stirrer system,

■ the flow against the membranes,

■ the primary flow profile generated by the stirrer system.

The resistance of the laminar boundary layer on the inner side of the hollow fiber membrane can be neglected due to the low inner diameter and the concomitant high flow velocities. Within this application the term "inner side of the hollow fiber membrane" denotes the side of the hollow fiber membrane which faces the storage container. The term "outer side of the hollow fiber membrane" denotes the side of the hollow fiber membrane which faces the cultivation vessel. The total mass transfer resistance is thus a series resistance to which mainly the resistance within the membrane and the resistance of the outer laminar boundary layer contribute (Rehm, et. al., Biotechnology - volume 3: Bioprocessing, VCH Weinheim, 1993). The total mass transfer coefficient k results from the reciprocal total mass transfer resistance and can be related to the surface area by multiplication with the volume-specific surface a of the hollow fiber dialysis module (ka value).The following Equation 8 can be used to describe the concentration time courses for the balance spaces reactor and storage container:

$$\frac{dc_R}{dt} = ka \cdot (c^* - c_R) \qquad \text{(Equation 8)}.$$

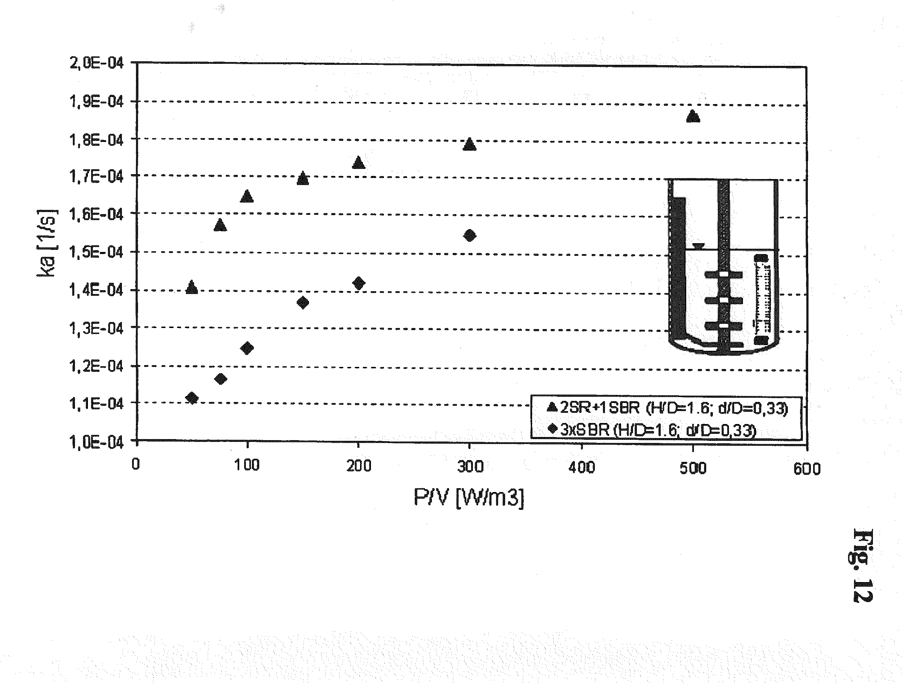

In general submerse gassed cultivation vessels are used in cell culture. In these cases a one-stage or two-stage axially-conveying stirrer system is mainly used. This generates a flow profile which is essentially parallel to the rotary shaft. Thus, in the arrangement as shown in Figure 12 with the dialysis module parallel to the rotary shaft a direct flow against the dialysis module is not achieved. This has a disadvantageous effect on the mass transport in the dialysis module (wider outer laminar boundary layer on the fiber surface).

A direct tangential or radial flow against the dialysis membrane has an advantageous effect which can for example be achieved by a standard anchor impeller. This impeller generates a flow which is directed directly onto the dialysis module or modules in the reactor and thus reduces the laminar boundary layer on the surface of the dialysis module(s). This simple radial flow is, however, disadvantageous for the other basic technical process functions in particular with regard to mixing the reactor and mass transfer especially in submersed gassed reactors. The gas can be introduced into the cultivation vessel e.g. via a pipe sparger or a ring sparger.

The stirrer system as reported herein can, in comparison to other stirrer systems, be used to more rapidly mix cultivation medium for example in order to introduce correcting agents such as acids or bases via the liquid surface, to reduce foam formation and in dialysis methods to reduce biofowling and increase the mass transfer rate by the direct orthogonal flow against the dialysis module.

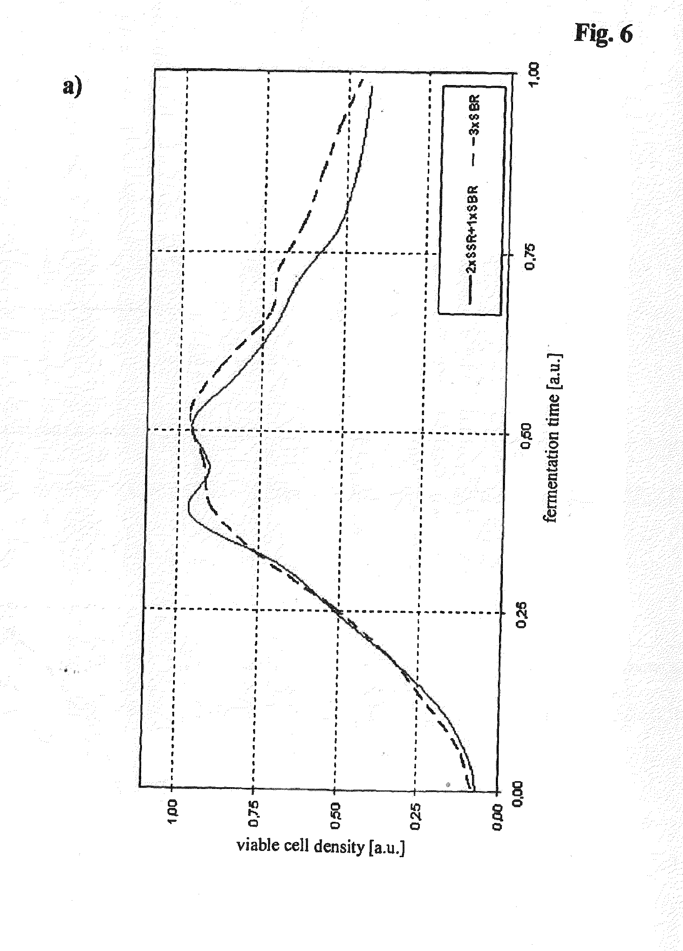

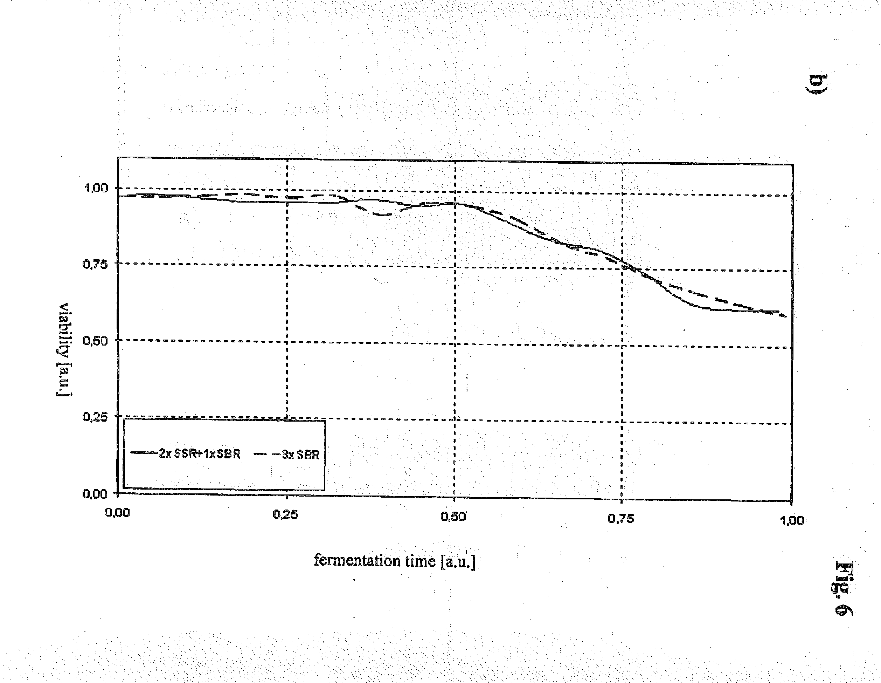

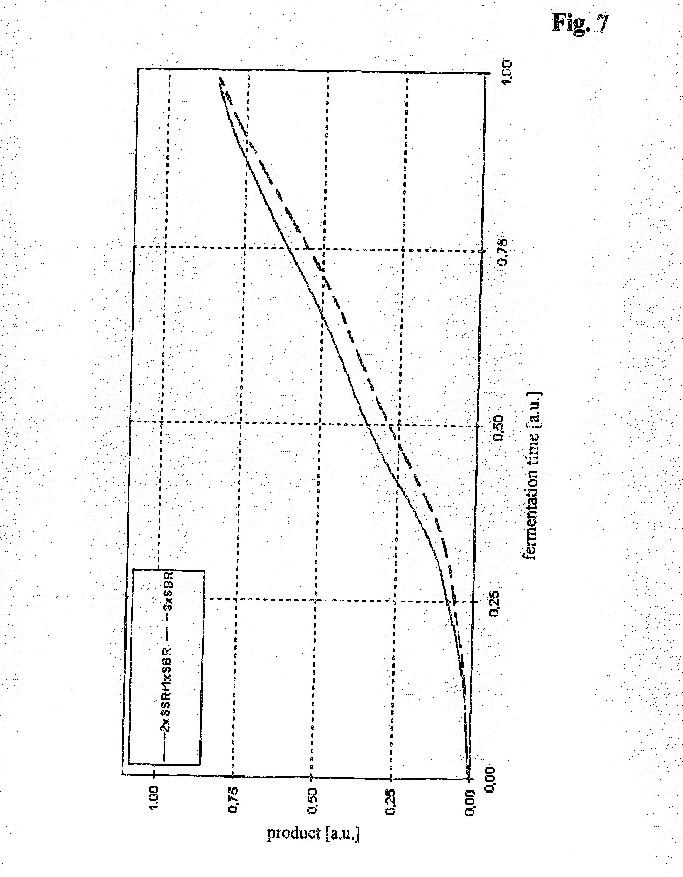

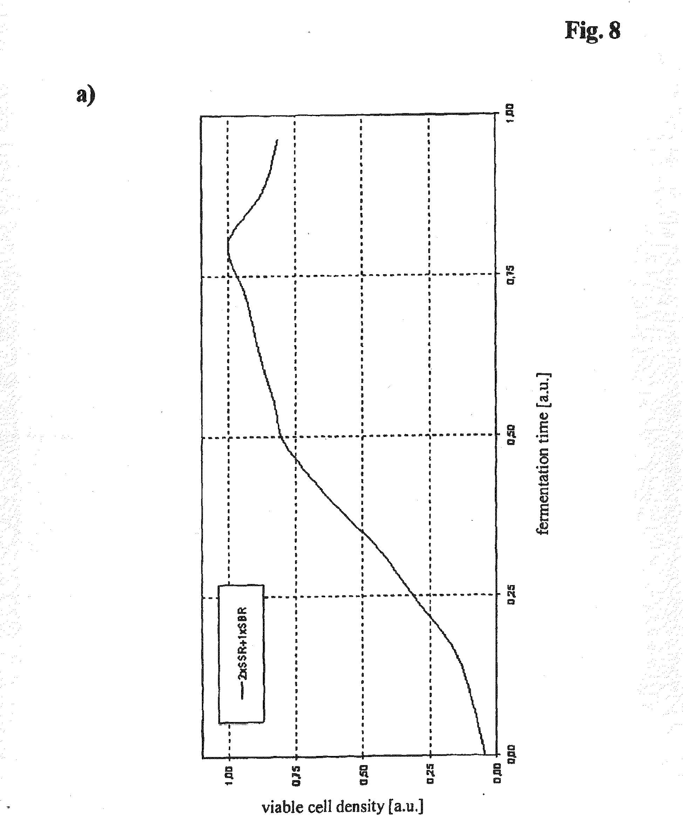

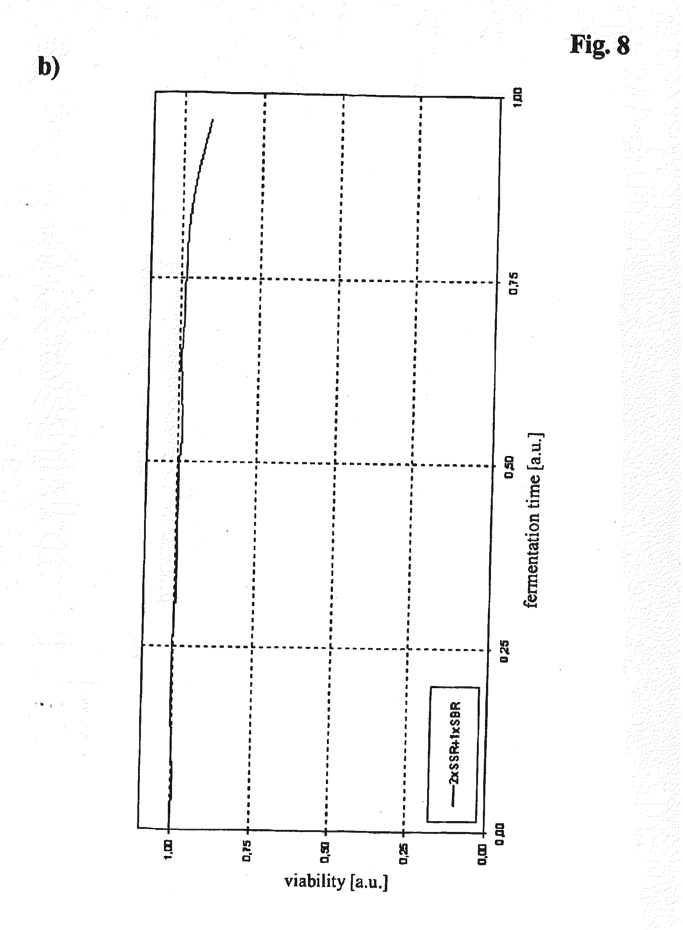

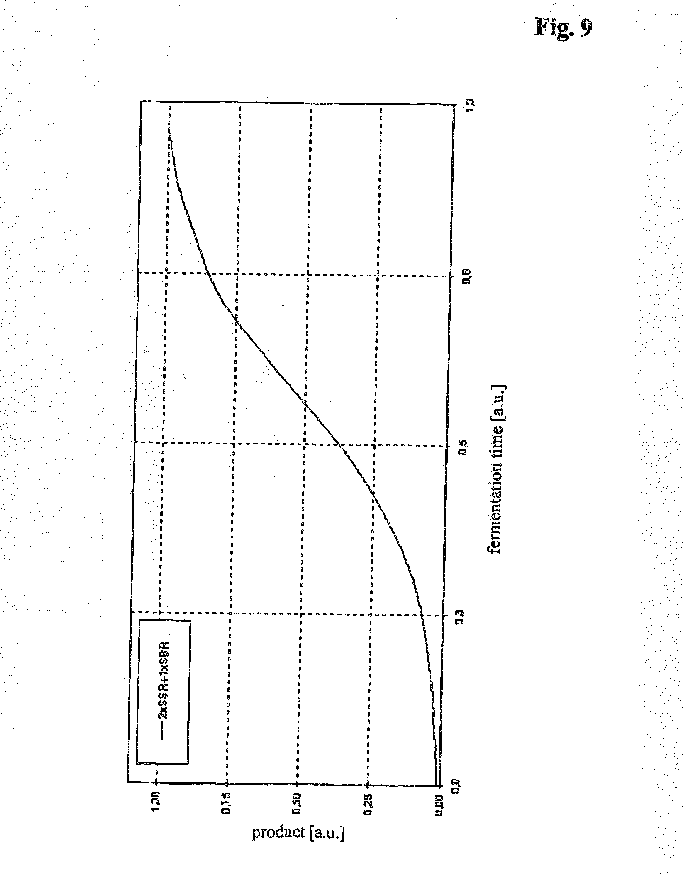

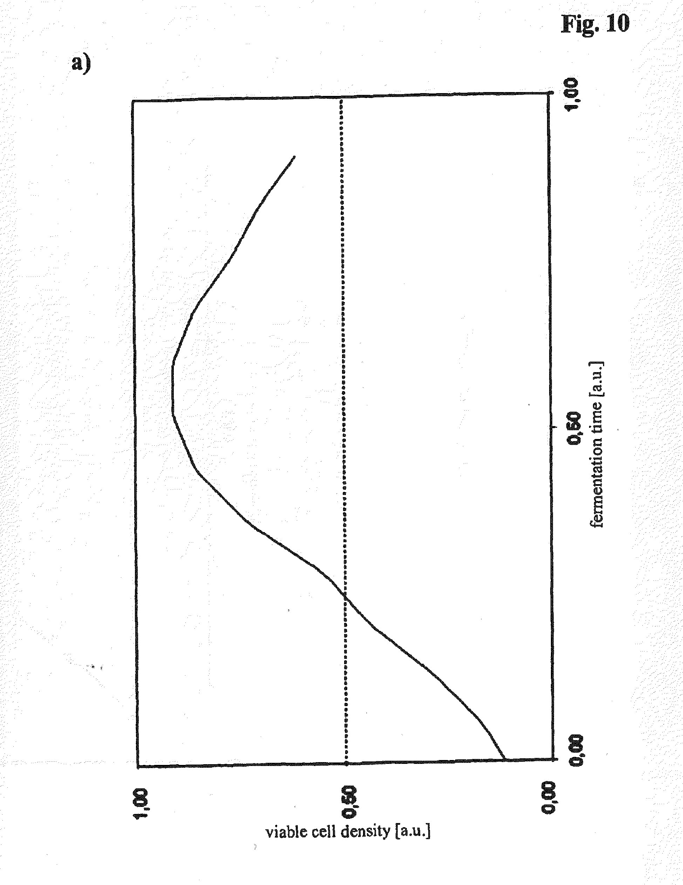

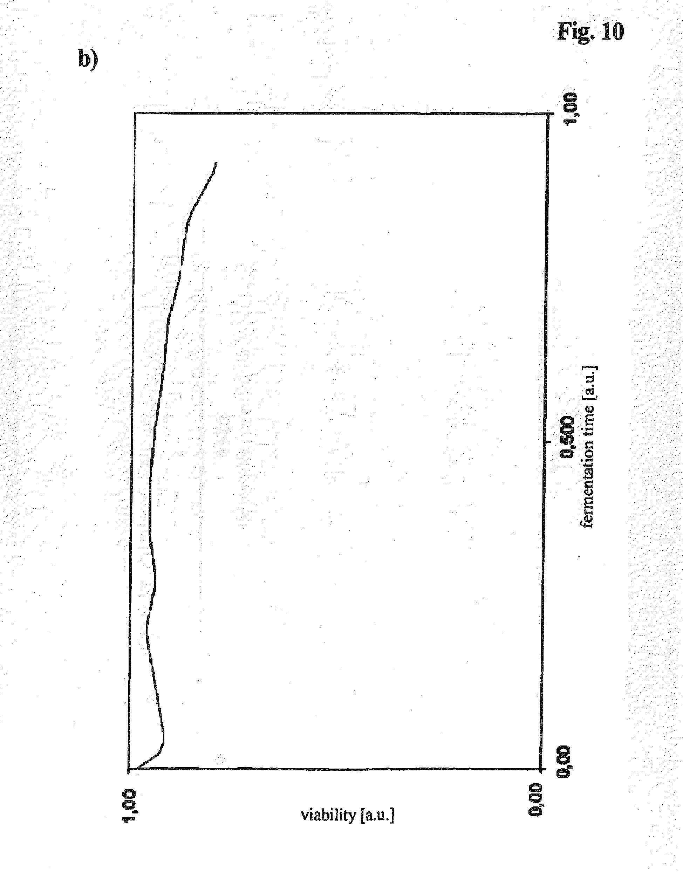

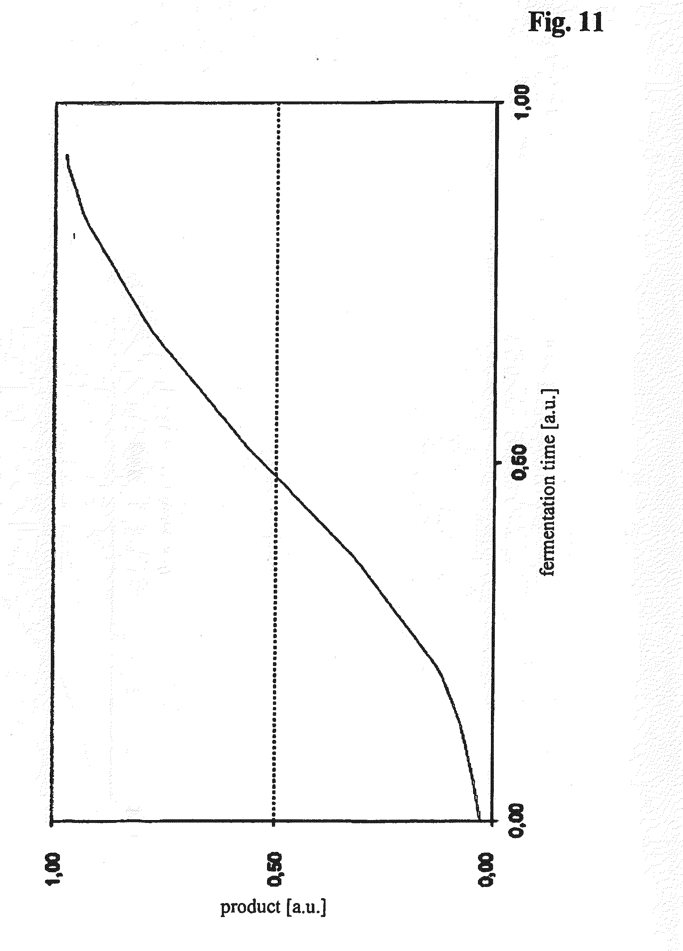

The use of the stirrer system according to the invention in the cultivation of animal cells which produce an antibody against IGF-1R or CD20 or HER2 was shown as an example (see WO 2004/087756; see WO 2007/045465, WO 2007/115814 and WO 2005/044859; see WO 99/057134 and WO 92/022653). This does not represent a limitation of disclosure but rather only serves to illustrate the invention. As can be seen from Figures 6 to 11 the use of a stirrer system as reported herein shows similar time courses for live cell density, viability and product formation when compared with a different stirrer system (such as e.g. three inclined-blade stirrers).

The abbreviations used in this application have the following meanings (see also Figure 1 a):

b: width of the blades of the radially-conveying element
d: agitator total outer diameter
$d_w$: diameter of the shaft
h: height of the stirrer blades of the radially-conveying element
$h_B$: height of an axially-conveying element
$\Delta h$: height difference of two conveying elements
l: length of the stirrer blades of an axially-conveying element
$\alpha$: blade pitch of the blades of an axially-conveying element
z: number of stirrer blades per stirrer
$d_i$: inner distance between the stirrer blades of the radially-conveying element
D: cultivation vessel inner diameter
H: filling height of the cultivation vessel.

In one embodiment the ratio of the height difference ($\Delta$h) of two conveying elements to the cultivation vessel diameter (D) is at least 0.75. Herein is reported as an aspect the use of the stirrer system as reported herein for the culturing of cells for the production of polypeptides or antibodies. In one embodiment the culturing is a dialysis. In a further embodiment the culturing is carried out in a submersed gassed stirred tank cultivation vessel. In another embodiment the cell is a eukaryotic cell, in another embodiment a mammalian cell. In yet a further embodiment the cell is a CHO cell, a BHK cell, an NS0 cell, a COS cell, a PER.C6 cell, a Sp2/0 cell or a HEK 293 cell. In one embodiment the cell is selected from Arthrobacter protophormiae, Aspergillus niger, Aspergillus oryzae, Bacillus amyloliquefaciens, Bacillus subtilis, BHK cells, Candida boidinii, Cellulomonas cellulans, Corynebacterium lilium, Corynebacterium glutamicum, CHO cells, E.coli, Geobacillus stearothermophilus, H. polymorpha, HEK cells, HeLa cells, Lactobacillus delbruekii, Leuconostoc mesenteroides, Micrococcus luteus, MDCK cells, Paenebacillus macerans, P. pastoris, Pseudomonas species, S. cerevisiae, Rhodobacter species, Rhodococcus erythropolis, Streptomyces species, Streptomyces anulatus, Streptomyces hygroscopicus, Sf-9 cells, and Xantomonas campestris. In yet a further embodiment the antibody is an antibody against CD19, CD20, CD22, HLA-DR, CD33, CD52, EGFR, G250, GD3, HER2, PSMA, CD56, VEGF, VEGF2, CEA, Lewis Y antigen, IL-6 receptor or IGF-1 receptor.

The following example and figures are provided to illustrate the subject matter of the invention. The protective scope is defined by the attached patent claims. It is clear that modifications can be made on the subject matter of the disclosed methods without departing from the subject matter of the invention.

## Description of the figures

| | |
|---|---|
| **Figure 1** | a) Diagram of the stirrer system as reported herein in a reactor in which D = container inner diameter, d = stirrer system outer diameter, $d_{sp}$ = diameter of the gas distributer (outlet openings), H = container filling level, $H_B$ = container height, $h_{sp}$ = installation height of the gas distributor, $h_{1m}$ = installation height of the lower stirrer element, $h_{2m}$ = distance between stirrer element 1 and stirrer element 2, $h_{3m}$ = distance between stirrer element 2 and stirrer element 3, $b_d$ = distance of the baffle from the container wall, w = width of the blade of the baffle; b) diagram of a standard disk stirrer in which: d = stirrer outer diameter, b = stirrer blade width, h = stirrer blade height; c) diagram of an inclined-blade stirrer in which: $\alpha$ = pitch of the stirrer blade, 1 = stirrer blade length, h = $h_{SB}$ = (projected stirrer blade height). |
| **Figure 2** | Diagram of the power coefficient Ne of various stirrer systems as a function of the Reynolds number; SSR = standard disk stirrer; SBR = inclined-blade stirrer. |
| **Figure 3** | Diagram of the mixing time for a degree of intermixing of 95 % as a function of the power input for various stirrer systems. |
| **Figure 4** | Comparison of the mixing coefficients $C_H$ for various stirrer systems as a function of the Reynolds number; SSR = standard disk stirrer; SBR = inclined-blade stirrer. |
| **Figure 5** | Dependency of the reference flake diameter $d_{VF}$ on the volume-specific power input and the stirrer system; SSR = standard disk stirrer; SBR = inclined-blade stirrer. |
| **Figure 6** | Standardized time course of the live cell density (a) and the viability (b) as a function of the fermentation time and the stirrer system used to culture an anti-IGF-1R antibody-producing cell line. |
| **Figure 7** | Standardized time course of the product concentration as a function of the fermentation time and of the stirrer system used to culture an anti-IGF-IR antibody-producing cell line. |
| **Figure 8** | Standardized time course of the live cell density (a) and the viability (b) as a function of the fermentation time using a stirrer system as reported herein to culture an anti-CD20 antibody-producing cell line. |
| **Figure 9** | Standardized time course of the product concentration as a function of the fermentation time and the stirrer system used to culture an anti-CD20 antibody-producing cell line. |
| **Figure 10** | Standardized time course of the live cell density (a) and the viability (b) as a function of the fermentation time using a stirrer system as reported herein to culture an anti-HER2 antibody-producing cell line. |
| **Figure 11** | Standardized time course of the product concentration as a function of the fermentation time and the stirrer system used to culture an anti-HER2 antibody-producing cell line. |
| **Figure 12** | Mass transfer coefficient in the dialysis as a function of the specific power input. |
| **Figure 13** | Schematic diagram of a device for dialysis cultivation. |
| **Figure 14** | Schematic diagram of the concentration gradients on the hollow fibers of the dialysis module. |

### Example 1

**Cultivation devices**

All investigations were carried out in a 300 1 Plexiglas® model container (referred to as DN 640 in the following) or in the production reactors themselves. The dialysis investigations were carried out in a 100 1 Plexiglas® model container (referred to as DN 440 in the following).

### Example 2

**Power input**

The power input of different stirrer was determined by measuring the torque on the rotary shaft. A data processing system model GMV2 together with the torque sensor model DRFL-II-5-A (both from the "ETH Messtechnik" Company, Gschwend, Germany) were used to record the torque. For each stirrer system the torque was firstly recorded at various revolution speeds in the unfilled state ($M_{empty}$) and subsequently by means of a triplicate determination in the filled state ($M_{load}$) according to Equation 9:

$$M = M_{load} - M_{empty} \qquad \text{(Equation 9).}$$

Afterwards the corresponding Newton number (Ne number) and the Reynolds's number (Re number) were calculated for each point. Since the Newton numbers for a stirrer system become constant in the turbulent flow region, the calculated Newton numbers were subsequently averaged in this region (Uhl, V.W. and Gray, J.B., Mixing Theory and Practice, Academic Press, 1966). This mean represents the total Newton number of the respective stirrer.

### Example 3

**Homogenization**

The homogenization was determined using the color change method as well as using the conductivity method.
The color change method is based on the decolorization of a starch solution stained with iodine-potassium iodide by addition of sodium thiosulfate (I, KI, starch, $Na_2S_2O_3$ obtained from the Carl Roth GmbH & Co KG Company, Karlsruhe, Germany). A one molar sodium thiosulfate solution and a one molar iodine-potassium iodide solution (Lugol's solution) as well as a starch solution at a concentration of 10 g/l were used as the starting solutions. In correspondence with the conductivity experiments, at least four speed steps were examined per stirrer (quadruplicate determinations per speed step) in which a maximum of four experiments were carried out per container filling amount. In each case the starch solution was added once per filling of the container. For each individual measurement the corresponding volume of the iodine-potassium iodide solution was firstly added and subsequently the sodium thiosulfate was added. The mixing time has been determined manually from the time point at which the sodium thiosulfate was added and one second was subtracted in each case in order to take the addition time into consideration. After completion of the measurement the container filling volume was titrated (neutralized) with iodine-potassium iodide in order to compensate for the excess of the previously added sodium thiosulfate.
In the conductivity method the mixing time is defined as the time from addition of an electrolyte solution to the time at which the measured conductivity fluctuations for the last time exceed a tolerance range of ± 5 % around the conductivity values which are reached in a stationary state. If several probes are used, the longest detected mixing time in each case is regarded as representative for the entire system.
A 30 % (w/v) NaCl solution (NaCl crystalline, Merck KGaA Company, Darmstadt, Germany) was used as an electrolyte solution to determine the mixing time by the conductivity method. This was added in pulses onto the liquid surface at the rotary shaft of the stirrer and the volume per addition was selected such that the jumps in conductivity which resulted in a stationary state did not exceed 200 mS/cm.
For each stirrer at least four speed steps were examined. The mixing time was determined at least eight times per speed step and these eight values were averaged. The mixing coefficient of the respective stirrer systems is given as the mean of the mixing coefficients averaged per speed step. The conductivity was in each case measured by three 4-pole conductivity probes (TetraCon, WTW Company, Weilheim) at various radial or axial positions in the container. The conductivity signals were read out online via the measuring amplifier that was used (Cond813, Knick "Elektronische Messgeräte GmbH & Co, KG" Company, Berlin, Germany). The measured values were stored online and simultaneously for all probes by means of the software Paraly SW 109 (Knick "Elektronische Messgeräte GmbH & Co, KG" Company, Berlin, Germany) at a sampling rate of 5 seconds. After the series of measurements was completed the data were evaluated separately for each probe.

### Example 4

### Shear stress

A model particle system, the blue clay polymer flake system, was used to determine shear stress. This is a model particle system consisting of a cationic polymer (Praestol BC 650) and a clay mineral (blue clay) which is placed in the vessel. A flocculation reaction is started by adding Praestol BC 650 which generates flakes of a defined size. These flakes are subsequently broken up by the mechanical and hydrodynamic stress of the stirrer system. In the case of bubble-gassed systems they are additionally broken up by the energy dissipation when the bubbles are formed and burst. The average particle diameter of the model particle system was used as a measured variable to characterize the shear stress. In this case the change in the particle size was measured in situ by a Focused Beam Reflectance Measurement Probe from the Mettler Toledo Company (referred to as FBRM® in the following). The rates of change in particle size that were determined are a measure for the shear stress prevailing in the model system. The gradient of the rate of change of particle size becomes smaller during the course of the experiment but no equilibrium state is formed (particle comminution down to a diameter of the blue clay primary particles of $\approx 15\ \mu m$). For this reason an end flake diameter $d_{P50}$' for the blue clay polymer flake system was determined according to the following criterion (Equation 10):

$$\frac{d(d_{P50})}{dt} \leq 0.0055\,[\mu m/s] \quad \rightarrow \quad d_{P50} = d_{P50}' \qquad \text{(Equation 10).}$$

In order to ensure comparability of the end flake diameters at different power inputs and between different stirrers, the reference flake diameter was calculated as follows (Equations 11 to 13):

$$d_{VF} = m \cdot d_{P50}' - b \qquad \text{(Equation 11)}$$

$$m = 1{,}3 \cdot 10^{-6} \cdot \left(\frac{P}{V}\right)^2 + 1{,}37 \cdot 10^{-3} \cdot \left(\frac{P}{V}\right) + 2{,}46 \qquad \text{(Equation 12)}$$

$$b = 8{,}12 \cdot 10^{-5} \cdot \left(\frac{P}{V}\right)^2 + 6{,}48 \cdot 10^{-3} \cdot \left(\frac{P}{V}\right) + 76{,}9 \qquad \text{(Equation 13).}$$

Table 3: Substances used to determine the particle stress (concentrations are based on the container fill volume).

| Ingredient | Concentration | Manufacturer |
|---|---|---|
| Wittschlicker blue clay | 5 g/l | Braun Tonbergbau Co., Germany |
| Praestol 650 BC (solution 2 g/l) | 5 ml/l | Stockhaus GmbH & Co. KG, Krefeld, Germany |
| NaCl | 1 g/l | Merck KGaA, Darmstadt, Germany |

(continued)

| Ingredient | Concentration | Manufacturer |
|---|---|---|
| $CaCl_2$ (solution 30 g/l) | -- | Carl Roth GmbH & Co.KG, Karlsruhe, Germany |

Firstly the 100 l model container was filled with a corresponding volume (H/D ratio) of completely demineralized water (VE water) and maintained at a temperature of 20°C. Subsequently the conductivity was adjusted to a value of 1000 μS/cm by titration with a $CaCl_2$ solution. The conductivity was measured by a 4-pole conductivity probe (probe: TetraCon, WTW Co. Weilheim; measuring amplifier: Cond813, Knick "Elektronische Messgeräte GmbH & Co, KG" Company, Berlin, Germany). Afterwards the blue clay and the NaCl were added in appropriate amounts to the solution. Subsequently a homogenization phase took place at the highest speed with a duration of at least 20 minutes. The FBRM® probe (FBRM® Lasentec® D600L, Mettler-Toledo GmbH Co., Giessen, Germany) was mounted in the container perpendicular from above (immersion depth 300 mm) at a radial distance of 70 mm to the wall. The flocculation reaction was subsequently started by adding Praestol 650 BS at a defined speed. The measured values were recorded online by means of the program data acquisition control interface version 6.7.0 (Mettler-Toledo GmbH, Giessen, Germany). The reference flake diameter was determined from the measurement data. At least three power inputs were measured for each stirrer. In each case three measurements were carried out per power input.

## Example 5

### Dialysis (mass transfer liquid - liquid)

A NaCl solution (NaCl crystalline, Merck KGaA Company, Darmstadt, Germany) was used as a tracer substance to determine the concentration half-life of the module (DIADYN-DP 070 F1 OL; MICRODYN-NADIR GmbH Company, Wiesbaden, Germany) in relation to the stirrer system that was used and the volume-specific power input. The tracer substance was adjusted in the storage container at the start of each experimental run to a base-line conductivity of 1500 μS/cm. The reactor was filled with completely demineralized water for each experimental run. The filling volume of the reactor was 100 l (H/D = 1.6) and that of the storage container was 400 l (H/D = 2.0) and both containers were maintained at a temperature of 20°C at the start of each experiment. The conductivity in both containers was measured by a 4-pole conductivity probe (probe: TetraCon, WTW Co. Weilheim, Germany; measuring amplifier: Cond813, Knick "Elektronische Messgeräte GmbH & Co, KG" Company, Berlin, Germany). The sampling rate of the measurement amplifiers that were used was 5 seconds and the measurement values were stored online and simultaneously for all probes by means of the software Paraly SW 109 (Knick "Elektronische Messgeräte GmbH & Co, KG" Company, Berlin, Germany). The NaCl solution was circulated by means of a peristaltic pump between supply container and dialysis module (housing pump 520 U, Watson-Marlow GmbH, Company, Rommerskirchen, Germany) at a constant flow rate of 2.1 l/min. For the evaluation, probe 1 was used as a reference probe for the reactor and probe 3 was used as a reference probe for the storage container. The data of these two probes were evaluated by an evaluation routine. In each case at least six different power inputs in the reactor were investigated per stirrer.

In order to compare the mass transfer characteristics determined by means of the NaCl solution, additional measurements were carried out with a glucose solution as tracer substance. The experimental setup was not changed for this. A defined glucose concentration (glucose solid, Merck KGaA Company, Darmstadt, Germany) at a concentration of 3 g/l was provided in the storage container. The glucose concentration was determined manually and simultaneously for the storage container and reactor at a time interval of 10 minutes by means of a blood sugar measuring instrument (ACCU-CHEK® Aviva, Roche Diagnostics GmbH Company, Mannheim, Germany).

## Example 6

### Anti-IGF-1R antibody

The cell line secreting anti-IGF-1R antibodies was produced and cultured according to the data published in the International Patent Applications WO 2004/087756, WO 2007/045465 and WO 2007/115814 and by means of generally known methods.

### Example 7

**Anti-CD20 antibody**

The cell line secreting anti-CD20 antibodies was produced and cultured according to the data published in the International Patent Application WO 2005/0044859 and by means of generally known methods.

### Example 8

**Anti-HER2 antibody**

The cell line secreting anti-HER2 antibodies was produced and cultured according to the data published in the International Patent Applications WO 92/022653 and WO 99/057134 and by means of generally known methods.

**Claims**

1. Method for culturing mammalian cells, **characterized in that** the mammalian cells are cultured in a device comprising a stirrer system and a cultivation vessel,

    wherein the stirrer system is consisting of two radially-conveying stirrer elements and one axially-conveying stirrer element arranged above one another on a vertical stirrer shaft,
    wherein the axially-conveying stirrer element is arranged above the radially-conveying stirrer elements.

2. Method for producing a polypeptide comprising the following steps:

    a) culturing a mammalian cell comprising a nucleic acid encoding the polypeptide in a device comprising a stirrer system and a cultivation vessel,

        wherein the stirrer system is consisting of two radially-conveying stirrer elements and one axially-conveying stirrer element arranged above one another on a vertical stirrer shaft,
        wherein the axially-conveying stirrer element is arranged above the radially-conveying stirrer elements,

    b) recovering the polypeptide from the cultivation medium or from the cells,
    c) purifying the polypeptide and thereby producing the polypeptide.

3. The method according to any one of claims 1 to 2, **characterized in that** the stirrer system has when used in the cultivation vessel comprising a cultivation medium a Newton number of from 5.5 to 8.0 at a Reynolds number of from $5*10^4$ to $5*10^5$.

4. The method according to any one of the claims 1 to 3, **characterized in that** the stirrer system when used in the cultivation vessel comprising a cultivation medium has a mixing time $\theta_{0.95}$ of about 20 seconds at a power input of about 0.05 W/kg and of about 10 seconds at a power input of about 0.3 W/kg.

5. The method according to any one of claims 1 to 4, **characterized in that** the stirrer system is consisting of two disk stirrers or two Rushton turbines as radially-conveying stirrer elements and one inclined-blade stirrer as an axially-conveying stirrer element

6. The method according to any one of claims 1 to 5, **characterized in that** the radially-conveying stirrer elements have between 2 and 8 stirrer blades and the axially-conveying stirrer element has between 2 and 10 stirrer blades.

7. The method according to any one of the claims 1 to 6, **characterized in that** all conveying elements have the same diameter d.

8. The method according to any one of claims 1 to 7, **characterized in that** the ratio of the diameter d of the stirring elements to the diameter D of the cultivation vessel is in the range of from 0.32 to 0.35.

9. The method according to any one of claims 1 to 8, **characterized in that** the pitch of the stirrer blades of the axially-conveying stirrer element is between 10° and 80° relative to the stirrer shaft.

**Patentansprüche**

1. Verfahren zum Kultivieren von Säugetierzellen, **dadurch gekennzeichnet, dass** die Säugetierzellen in einer Vorrichtung kultiviert werden, die ein Rührsystem und ein Kultivierungsgefäß aufweist,

   wobei das Rührsystem aus zwei radialfördernden Rührelementen und einem axialfördernden Rührelement besteht, die an einer vertikalen Rührwelle übereinander angeordnet sind,
   wobei das axialfördernde Rührelement oberhalb der radialfördernden Rührelemente angeordnet ist.

2. Verfahren zur Produktion eines Polypeptids mit den folgenden Schritten:

   a) Kultivieren von Säugetierzellen, die eine das Polypeptid kodierende Nucleinsäure aufweisen, in einer Vorrichtung, die ein Rührsystem und ein Kultivierungsgefäß aufweist, wobei das Rührsystem aus zwei radialfördernden Rührelementen und einem axialfördernden Rührelement besteht, die an einer vertikalen Rührwelle übereinander angeordnet sind,
   wobei das axialfördernde Rührelement oberhalb der radialfördernden Rührelemente angeordnet ist,
   b) Gewinnen des Polypeptids aus dem Kultivierungsmedium oder aus den Zellen,
   c) Reinigen des Polypeptids und dadurch Produzieren des Polypeptids.

3. Verfahren nach einem der Ansprüche 1 bis 2, **dadurch gekennzeichnet, dass** das Rührsystem, wenn es in dem Kultivierungsgefäß verwendet wird, das ein Kultivierungsmedium aufweist, eine Newton-Zahl von 5,5 bis 8,0 bei einer Reynolds-Zahl von $5*10^4$ bis $5*10^5$ hat.

4. Verfahren nach einem der Ansprüche 1 bis 3, **dadurch gekennzeichnet, dass** das Rührsystem, wenn es in dem Kultivierungsgefäß verwendet wird, das ein Kultivierungsmedium aufweist, eine Mischzeit $\Theta_{0,95}$ von ungefähr 20 Sekunden bei einer Leistungszufuhr von ungefähr 0,05W/kg und von ungefähr 10 Sekunden bei einer Leistungszufuhr von ungefähr 0,3W/kg hat.

5. Verfahren nach einem der Ansprüche 1 bis 4, **dadurch gekennzeichnet, dass** das Rührsystem aus zwei Scheibenrührern oder zwei Rushton-Turbinen als radialfördernde Rührelemente und einem Rührer mit einem geneigten Blatt als ein axialförderndes Rührelement besteht.

6. Verfahren nach einem der Ansprüche 1 bis 5, **dadurch gekennzeichnet, dass** die radialfördernden Rührelemente zwischen 2 und 8 Rührblätter haben und das axialfördernde Rührelement zwischen 2 und 10 Rührblätter hat.

7. Verfahren nach einem der Ansprüche 1 bis 6, **dadurch gekennzeichnet, dass** alle Förderelemente den gleichen Durchmesser d haben.

8. Verfahren nach einem der Ansprüche 1 bis 7, **dadurch gekennzeichnet, dass** das Verhältnis des Durchmessers d der Rührelemente zum Durchmesser D des Kultivierungsgefäßes im Bereich von 0,32 bis 0,35 ist.

9. Verfahren nach einem der Ansprüche 1 bis 8, **dadurch gekennzeichnet, dass** die Neigung der Rührblätter des axialfördernden Rührelements zwischen 10° und 80° relativ zur Rührwelle ist.

**Revendications**

1. Procédé pour cultiver des cellules de mammifère, **caractérisé en ce que** les cellules de mammifère sont cultivées dans un dispositif comprenant un système d'agitateur et un récipient de culture,

   dans lequel le système d'agitateur consiste en deux éléments d'agitateur à transport radial et un élément d'agitateur à transport axial disposés les uns au-dessus des autres sur un arbre d'agitateur vertical,
   dans lequel l'élément d'agitateur à transport axial est disposé au-dessus des éléments d'agitateur à transport radial.

**2.** Procédé pour produire un polypeptide, comprenant les étapes suivantes :

a) culture d'une cellule de mammifère comprenant un acide nucléique codant pour le polypeptide dans un dispositif comprenant un système d'agitateur et un récipient de culture,

le système d'agitateur consistant en deux éléments d'agitateur à transport radial et un élément d'agitateur à transport axial disposés les uns au-dessus des autres sur un arbre d'agitateur vertical,
l'élément d'agitateur à transport axial étant disposé au-dessus des éléments d'agitateur à transport radial,

b) la récupération du polypeptide à partir du milieu de culture ou à partir des cellules,
c) la purification du polypeptide et, ainsi, la production du polypeptide.

**3.** Procédé suivant l'une quelconque des revendications 1 et 2, **caractérisé en ce que** le système d'agitateur a, lorsqu'il est utilisé dans le récipient de culture comprenant un milieu de culture, une valeur Newton de 5,5 à 8,0 à un indice Reynolds de $5*10^4$ à $5*10^5$.

**4.** Procédé suivant l'une quelconque des revendications 1 à 3, **caractérisé en ce que** le système d'agitateur, lorsqu'il est utilisé dans le récipient de culture comprenant un milieu de culture, a un temps de mélange $\theta_{0,95}$ d'environ 20 secondes à une puissance émise d'environ 0,05 W/kg et d'environ 10 secondes à une puissance émise d'environ 0,3 W/kg.

**5.** Procédé suivant l'une quelconque des revendications 1 à 4, **caractérisé en ce que** le système d'agitateur consiste en deux agitateurs à disque ou deux turbines Rushton comme éléments d'agitateur à transport radial et un agitateur à pale inclinée comme élément d'agitateur à transport axial.

**6.** Procédé suivant l'une quelconque des revendications 1 à 5, **caractérisé en ce que** les éléments d'agitateur à transport radial comportent 2 à 8 pales d'agitateur et l'élément d'agitateur à transport axial comporte 2 à 10 pales d'agitateur.

**7.** Procédé suivant l'une quelconque des revendications 1 à 6, **caractérisé en ce que** tous les éléments de transport ont le même diamètre d.

**8.** Procédé suivant l'une quelconque des revendications 1 à 7, **caractérisé en ce que** le rapport du diamètre d des éléments d'agitation au diamètre D du récipient de culture est compris dans l'intervalle de 0,32 à 0,35.

**9.** Procédé suivant l'une quelconque des revendications 1 à 8, **caractérisé en ce que** le pas des pales d'agitateur de l'élément d'agitateur à transport axial est compris entre 10° et 80° par rapport à l'arbre d'agitateur.

Fig. 1

Fig. 1

**Fig. 1**

e)

$d_i$ – inner diameter

connector

stirrer blade

hub

shaft

shaft axis

inner edge of stirrer blade

outer edge of stirrer blade

f)

diameter

tip of stirrer blade

stirrer blade

shaft axis

hub

shaft

Fig. 2

EP 2 456 547 B2

Fig. 3

Fig. 4

Fig. 5

**Fig. 6**

a)

Fig. 6

**Fig. 7**

**Fig. 8**

a)

Fig. 8

Fig. 9

a)

Fig. 10

Fig. 10

Fig. 11

Fig. 12

EP 2 456 547 B2

**Fig. 13**

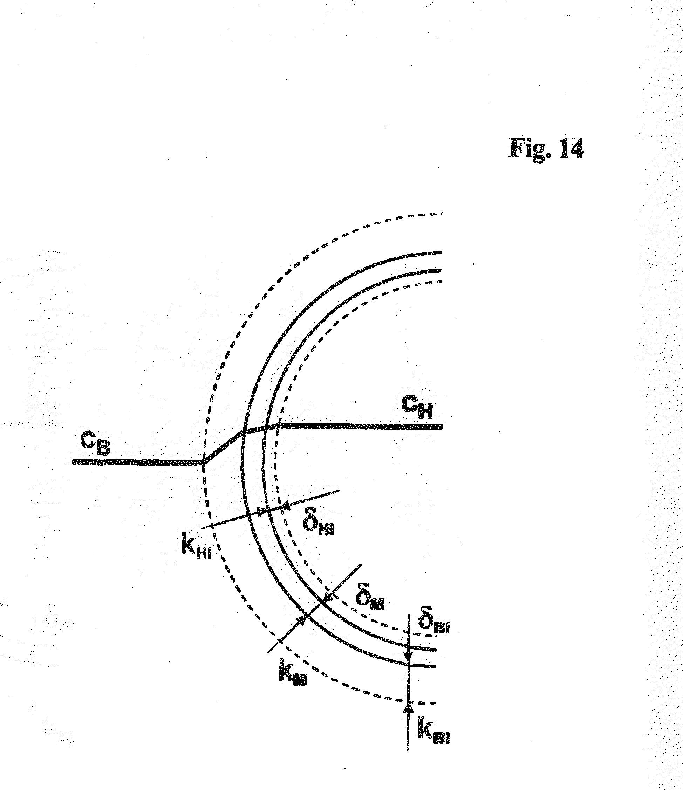

**Fig. 14**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5633165 A
- US 20040234435 A
- US 4438074 A
- US 20040087814 A
- US 5972661 A
- US 6250769 B
- US 5198156 A
- US 4779990 A

- WO 2004087756 A
- WO 2007045465 A
- WO 2007115814 A
- WO 2005044859 A
- WO 99057134 A
- WO 92022653 A
- WO 20050044859 A

**Non-patent literature cited in the description**

- **GLACKEN, M.W. et al.** *Trends Biotechnol.,* 1983, vol. 1, 102-108
- **VAN DER POL, L.A. ; TRAMPER, J.** *Enzyme Microb. Technol.,* 1995, vol. 17, 401-407
- **NIENOW, A.W.** *Cytotechnol.,* 2006, vol. 50, 9-33
- **CERVANTES, M.I. et al.** *Chem. Eng. Sci.,* 2006, vol. 61, 8075-8084
- **FRAHM, B. et al.** *Chem. Ing. Tech.,* 2007, vol. 79, 1052-1058
- **CHERRY, R.S. ; PAPOUTSAKIS, E.T.** *Biotechnol. Bioeng.,* 1988, vol. 32, 1001-1014
- **PAPOUTSAKIS, E.T. ; KUNAS, K.T. et al.** Advances in animal cell biology and technology for bioprocesses. Butterworth, 1989, 203-211
- **KUNAS, K.T. ; PAPOUTSAKIS, E.T.** *Biotech. Bioeng.,* 1990, vol. 36, 476-483
- **ZHANG, Z.B. ; THOMAS, C.R.** *Gen. Eng. Biotechnol.,* 1993, vol. 13, 19-29
- **FEDER, J. ; TOLBERT, W.R.** *Sci. Am.,* 1983, vol. 248, 24-31
- *Axial flow down-pumping agitators in biological processes,* April 2009, http://www.postmixing. com/mixing%20forum/Micro/Liq-Solid-Gas/down-pumpers.htm

- **FUJASOVA, M. et al.** *Chem. Eng. Sci.,* 2007, vol. 62, 1650-1669
- **PUTHLI, M.S. et al.** *Biochem. Eng. J.,* 2005, vol. 23, 25-30
- **HOUCINE, I. et al.** *Chem. Eng. Technol.,* 2000, vol. 23, 605-613
- **ZLOKARNIK, M.** Rührtechnik Theorie und Praxis. Springer Publishers, 1999
- **HENZLER, H.-J.** Homogenisieren: Referenz-Rührsysteme und Methoden zur Erfassung der Homogenisierungseigenschaften von Rührsystemen. *GVC Fachausschuß Mischvorgänge,* 1998
- **WOLLNY, S. ; SPERLING, R.** *Chem. Ing. Tec.,* 2007, vol. 79, 199-208
- **HENZLER, H.J. ; BIEDERMANN, A.** *Chem. Ing. Tec.,* 1996, vol. 68, 1546-1561
- Bioprocessing. **REHM.** Biotechnology. VCH, 1993, vol. 3
- **UHL, V.W. ; GRAY, J.B.** Mixing Theory and Practice. Academic Press, 1966